(19)

Europäisches
Patentamt
European
Patent Office
Office européen
des brevets

(11) **EP 3 021 142 A1**

(12) **EUROPEAN PATENT APPLICATION**

(43) Date of publication:
**18.05.2016 Bulletin 2016/20**

(21) Application number: **14194826.5**

(22) Date of filing: **25.11.2014**

(51) Int Cl.:
*G01T 7/00* *(2006.01)*        *A61B 6/00* *(2006.01)*
*G01N 23/04* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **08.09.2014 PL 40940814**

(71) Applicant: **Uniwersystet Slaski w Katowicach
40-007 Katowice (PL)**

(72) Inventor: **Bi kowski, Marcin
40-175 Katowice (PL)**

(74) Representative: **Rumpel, Alicja et al
Al. Smiglego-Rydza 29/11
93-281 Lodz (PL)**

(54) **Method of mapping distribution of physical parameters of a reference used in tests employing electromagnetic radiation**

(57)    The subject of the invention comprises of a method of mapping of distribution of reference physical parameters used in tests applying electromagnetic waves, in particular in planar or spatial tests of objects imagined using a computer tomograph, wherein the entire reference (1) or its fragments of components used in its design and forming determinants of its physical parameters are imaged by high-resolution scanning, that is, at least twice, preferably five times higher than the resolution in which the reference will be used in future studies and a collection of layered images of a reference or its fragment or component is obtained, on the basis of which, by reading information out of the image of the particular cross-section, material distribution and/or absorption coefficient distribution is determined directly, with the information about the absorption coefficient, together with coordinates for every voxel, which form so called spatial distribution of the absorption coefficient for the particular reference element are stored in a three-dimensional matrix, in electronic memory, with said information being used to calculate the correction coefficient, which defines for every voxel the deviation of parameters of the particular part of element of the reference from the theoretical value resulting from manufacturing assumptions, forming so called map of manufacturing precision, individual for the particular fragment or element of the reference, and then the individual manufacturing precision map for a part of the reference or its elements is written into a common file forming the manufacturing precision definition for the entire reference.

Fig. 1

EP 3 021 142 A1

**Description**

The subject of the invention comprises of mapping of physical parameters distribution of a reference used in tests employing electromagnetic waves, in particular in planar or spatial tests of objects imaged using a computer tomograph.

The X-ray computer tomography method comprises of recording so called tomographic projections which form the basis of computer reconstruction, aimed at obtaining two-dimensional images showing cross-sections of the studied object. These cross-sections may then be used as basis for computer reconstruction of a three-dimensional shape of the studied object. Because the basic application of computer tomography includes studies of human or animal body, in order to determine the condition of internal organs, computer tomography results are most often presented as transverse cross-sections or as three-dimensional reconstructions, in which the image is presented in so called Hounsfield scale. This scale defines the level of radiation absorption within the scanned object and is usually visualised as greyscale in which the lowest intensity, for example white colour, denotes the maximum and the lowest intensity, for example the black colour, denotes minimum absorption level of X-ray radiation in the given range, The absorption level value expressed in so called Hounsfield units for biological tissue was determined as an absolute reference value for properties of X-ray radiation absorption by water. The Hounsfield scale provides the options of imaging the given object at level adequate for imaging diagnostics. However, this method offers no possibility of determination of qualitative and absolute value of imaged density of the studied object and of practical evaluation of this value.

High-resolution computer tomography, namely microtomography or nanotomography does not use the Hunsfield scale, but instead a greyscale recorded in 256 (8-bit) or 65500M (16 bits unsigned short) shades of grey or a float value scale in the range from -32 000 to 32 000 (32 bits float) are used, they are still relative scales, not enabling quantitative determination of physical parameters.

Methods for determination of physical parameters of the studied obj ects imaged using a computer tomograph are known in the art. For example, patent PL213008 discloses a method of determination of physical density of the tested object imaged using computer tomograph and a phantom (reference) for implementation of the method. This solution had proposed a reference of physical density of an object imaged using a computer tomograph, in the shape of a batten made of material possibly not absorbing X-rays, with through, cylindrical openings made along its length, preferably seven parallel openings filled replacable with material with different levels of X-ray absorption, forming comparative determinants of the physical density category, preferably in the value range of 0 mg HA/ccm to 1200 mg HA/ccm.

Polish patent application P.409092 also discloses a method of determination of physical parameters of an object imaged using computer tomograph and a system for implementation of this method. The goal of this invention is to create the possibility of practical, quantitative determination of physical parameters, in particular of physical density and the X-ray absorption factor for the studied object using computer tomography, including microtomography and nanotomography. The system installed in this solution is characterised by the fact that the reference of the determined physical parameters is installed between the X-ray source and detector, preferably directly on the detector, outside the rotating platform of the tomograph where the scanned object is placed, in such a way that the X-ray image of the reference was contained in the projection image recorded during image scanning, where the reference is formed of a batten in which at least one opening running or not through the batten is provided, preferably a cylindrical opening, filled with material forming the determinant of the determined physical parameter, enabling direct or indirect determination of the physical parameter value within the scanned obj ect.

The solutions known in the art, including solutions indicated above, do not provide adequate accuracy, however, which results from impossibility of manufacturing an ideal reference. An ideal reference should comprise an error-free, precise reflection of physical parameters, the known values of which will be used for determination of so called calibration curve.

Meanwhile, in practice, even with maintaining high quality of materials, production processes and quality control, the obtained product, namely the reference, shows deviations from the assumed standard which are defined with accuracy related to the amount of material used for manufacturing (mass and volume), and not with precision referring to its uniform distribution within the reference area. During the manufacturing process of reference components, for example bars made of materials with various values of X-ray absorption coefficient, significant deviations from the assumed value of the net absorption coefficient occur. This is a characteristic for most references used in tomography or microtomography. Technological imperfections in reference design have not been included so far in further calculations and may distort the results of determined physical parameters of the tested object. This is the situation taking place in case of measurements with resolution similar to the size of grains used in reference design. In case of references used for bone tissue measurements, this is hydroxyapatite powder, in which the grain diameter equals 5 to even 30 micrometres, thus in case of measurements made at similar resolution, the spread of obtained results may be quite significant, resulting from lack of information about material distribution in the reference.

The aim of this intention is creating an opportunity of practical, quantitative and precise determination of not only the amounts of material used in reference design and ensuring the defined range of physical parameters, but also for precise measurements of the net distribution of this material, individually for every manufactured reference, and then recording an individual parameter correction card which may be used every time the reference is used in tests on an object using computer tomography, including microtomography.

This task was accomplished through development of a method of mapping distribution of physical parameters of the reference used in studies applying electromagnetic radiation, in particular in planar or spatial studies of objects imaged using a computer tomograph, the core of which resided in the fact that the reference, before it is applied in practice for determination of - using methods disclosed for example in the patent description of PL213008 or the Polish patent application P.409092 - physical parameters of an object imaged using computer tomography, is subjected to rigorous manufacturing control, which comprises of creation of a spatial, high-resolution map of deviations of physical parameter values from theoretically assumed values.

The method according to the invention is based on the fact that the entire reference or its parts or components designed for its construction and comprising determinants of its physical parameters are imaged by scanning at high resolution, that is at least twice (obtaining a smaller voxel size in the reconstructed data), preferably five times higher than the resolution in which the reference will be used in future studies and a collection of layered images of the reference is obtained, on the basis of which, through readout of information from the image of the particular cross-section, material and/or absorption coefficient distribution is determined, as well as the value of absorption coefficient and material grain size. Information about absorption coefficient together with coordinates for every voxel, which form so called spatial distribution of absorption coefficient for the particular reference part , is stored in a three-dimensional matrix, in the electronic memory, where the information is then used to calculate the correction parameter which defines deviation of parameters of the given reference part or element from the theoretical value of these parameters resulting from manufacturing assumptions, forming so called precision map of its manufacturing, individual for every reference part or element, and then the individual map of manufacturing precision for the reference part or its elements is saved in a common file forming the definition of manufacturing precision for the entire reference.

Preferably, the process of high-resolution scanning of the entire reference or its parts or elements used in its design is performed using imagining with polychromatic radiation in laboratory scanners equipped with an X-ray lamp, or using polychromatic radiation filtered in order to cut off low-energy photons, or monochromatised radiation or electromagnetic waves, in particular optical tomography, spectroscopic methods, including layer spectroscopy, light microscopy, confocal microscopy, ultrasound methods, acoustic and microwave methods, and most preferably using a synchrotron station with high-energy X-ray radiation, monochromatic or monochromatised.

Favourably, information about the absorption coefficient for every voxel is stored in a three-dimensional matrix, in a file or files, on a flash disk, SSD hard disk or HDD hard disk, in an electronic form.

Preferably, the correction coefficient is calculated as follows:

$$\text{correction coefficient} = Wp - Wz\,[\text{physical unit}]$$

$$\text{relative correction coefficient} = \frac{Wp - Wz}{Wp} * 100\,[\%]$$

where:

$W_p$ - theoretical value assumed at the stage of reference manufacturing
$W_z$ - measured value,

The value of the correction coefficient is used together with the reference in such a way that for every voxel, an appropriate value of correction coefficient for correction of physical parameter is taken into account during calculations of the calibration curve.

The correction coefficient and relative correction coefficient are related to two possibilities of correcting the reference indications. On the basis of standard values of the calculated correction coefficient, values for determinants of its physical parameters can be directly corrected. On the basis of the correction coefficient, the relative correction coefficient, expressed in percentage deviation from the theoretical value can also be calculated and used as a parameter.

Then the reference can be scanned using methods known, for example, from the patent disclosure PL213008 or the Polish patent application P.409092.

At the stage of performing calibration using methods known from the aforementioned inventions, for at least one reference layer values of image greyscale intensity are read in relative units, by reading the value of a pixel from the projection image, which reflects in the greyscale the intensity of X-ray radiation after passing through the scanner chamber and the reference, then the value is corrected using the correction coefficient calculated on the basis of theoretical value and value calculated during mapping, stored in the reference map. The calibration process is continued for thus corrected values in relative units, described i.e. in the aforementioned inventions known in the art.

The advantages of the method according to the invention include increased accuracy of the reference in the used method of quantitative measurements of physical parameters of the object tested using tomography. The higher quality resulting from use of the invention, namely mapping distribution of parameters of the manufactured reference, results not only from design assumptions and following the procedure of component manufacture and reference assembly, but first and foremost, every reference is supplied with a validation protocol after manufacture. The spatial information about the correction coefficient is provided for a part or, preferably, for the entire reference with the predetermined measurement accuracy resulting from imaging resolution at the stage of validation performed for the manufactured elements, a part of the reference or the entire reference.

Use of the reference enables - thanks to the known distribution of the correction coefficient - to correct the calibration curve used in calculations of parameters for the tested obj ect,

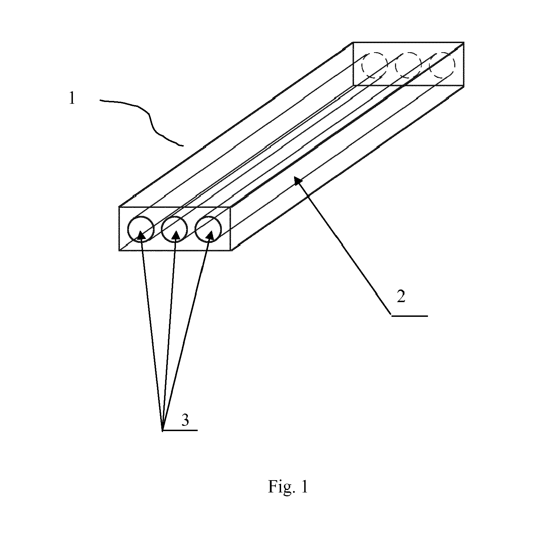

The subject of the invention is depicted as an embodiment in the drawing, in which fig. 1 - presents an axonometric view of the reference for determined physical parameters of the studied object, with three through openings filled with material forming the determinant of the determined physical parameter.

**Example 1.**

Example method of mapping distribution of physical parameters of the reference used in tests applying electromagnetic waves was performed using reference **1**, the main design element of which is the batten **2**, in which there are three cylindrical, through opening provided, along the length of the batten and parallel to one another **3**. Inside the openings, there are three bars placed tightly, having cylindrical shape and size matching the diameter of openings **3** in the batten **2**, that is, of 40 mm length and 0.5 mm in diameter. Batten **2** is made of polymethacrylate resin (PMMA), which has the mineral density of bone tissue equal to 0 mgHA/ccm. Bars are made of a mixture of an epoxide resin and synthetic hydroxyapatite HA. Weight ratios of the mixture of both materials are selected in such a way that the net mineral density in each of the bars was, respectively, 50, 200, 400 mgHA/ccm.

Every bar of reference **1** was imaged by scanning at a synchrotron station using monochromatic X-ray radiation, in such a way that the obtained image resolution was defined by the voxel size of $2x2x2\mu m$ and 20 000 cross-sections recorded in a single file were obtained, in such a manner, that the cross-section of the bar is visible in every cross-section. The measured value of mineral density have been determined on the basis of radiation absorption coefficient used in the synchrotron measurement and written into the file. Then, for every voxel of the image belonging to the bar, the value of mineral density was read from a file and compared with the theoretical value defined for the particular bar during its manufacturing, thus the correction factor was obtained, and after referring it to the theoretical value, it was obtained as relative correction coefficient. Calculations were performed according to the formulae:

$$\text{correction coefficient} = Wp - Wz \text{ [physical unit]}$$

$$\text{relative correction coefficient} = \frac{Wp - Wz}{Wp} * 100 \text{ [\%]}$$

where:

Wp - theoretical value assumed at the stage of reference manufacturing
$W_z$ - measured value,

and results of calculations for the selected voxel in each of the three bars are presented in Table 1.

Table 1

| mineral density | | correction factor | |
|---|---|---|---|
| theoretical value (production foredesign) | measured on the basis of high resolution image | | |
| mg/ccm | mg/ccm | mg/ccm | % |
| 50 | 40 | 10 | 20 |

(continued)

| mineral density | | correction factor | |
|---|---|---|---|
| theoretical value (production foredesign) | measured on the basis of high resolution image | | |
| 200 | 220 | -20 | -10 |
| 400 | 390 | 10 | 2,5 |

After the reference had been assembled, it was used in density measurements, where - using the known method - calibration curve was calculated, with values measured for bar areas in the reference before curve calculation were corrected using the determined correction factor.

## Claims

1. Method of mapping of distribution of reference physical parameters used in tests applying electromagnetic waves, in particular in planar or spatial tests of objects imagined using a computer tomograph, **characterised in that** the entire reference (1) or its fragments of components used in its design and forming determinants of its physical parameters are imagined by high-resolution scanning, that is, at least twice, preferably five times higher than the resolution in which the reference will be used in future studies and a collection of layered images of a reference or its fragment or component is obtained, on the basis of which, by reading information out of the image of the particular cross-section, material distribution and/or absorption coefficient distribution is determined directly, preferably distribution, value of absorption coefficient and material grain sizes, with the information about the absorption coefficient, together with coordinates for every voxel, which form so called spatial distribution of the absorption coefficient for the particular reference element are stored in a three-dimensional matrix, in electronic memory, with said information being used to calculate the correction coefficient, which defines for every voxel the deviation of parameters of the particular part of element of the reference from the theoretical value resulting from manufacturing assumptions, forming so called map of manufacturing precision, individual for the particular fragment or element of the reference, and then the individual manufacturing precision map for a part of the reference or its elements is written into a common file forming the manufacturing precision definition for the entire reference.

2. Method according to claim. 1 **characterised in that** the process of high-resolution scanning of the entire reference (1) or its parts or elements used in its design is performed using imagining with polychromatic radiation in laboratory scanners equipped with an X-ray lamp, or using polychromatic radiation filtered in order to cut off low-energy photons, or monochromatised radiation or electromagnetic waves, in particular optical tomography, spectroscopic methods, including layer spectroscopy, light microscopy, confocal microscopy, ultrasound methods, acoustic and microwave methods, and most preferably using a synchrotron station with high-energy X-ray radiation, monochromatic or monochromatised.

3. Method according to claim. 1 **characterised in that** information about the absorption coefficient for every voxel is stored in a three-dimensional matrix, in a file or files, on a flash disk, SSD hard disk or HDD hard disk, in an electronic form.

4. Method according to claim. 1 **characterised in that** correction coefficient is calculated in the following manner:

$$\text{correction coefficient} = Wp - Wz \, [\text{physical unit}]$$

$$\text{relative correction coefficient} = \frac{Wp - Wz}{Wp} * 100 \, [\%]$$

where:

Wp - theoretical value assumed at the stage of reference manufacturing
$W_z$ - measured value,

1

2

3

Fig. 1

**Europäisches Patentamt**
**European Patent Office**
**Office européen des brevets**

# EUROPEAN SEARCH REPORT

Application Number

EP 14 19 4826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAZARIAN A ET AL: "Quantitative micro-computed tomography: A non-invasive method to assess equivalent bone mineral density", BONE, PERGAMON PRESS., OXFORD, GB, vol. 43, no. 2, August 2008 (2008-08), pages 302-311, XP025671905, ISSN: 8756-3282, DOI: 10.1016/J.BONE.2008.04.009 [retrieved on 2008-04-30] * the whole document * | 1-4 | INV. G01T7/00 A61B6/00 G01N23/04 |
| A | Philipp Martin Krämer: "Simulationsgestützte Abschätzung der Genauigkeit von Messungen mit Röntgen-Computertomographie" In: "Simulationsgestützte Abschätzung der Genauigkeit von Messungen mit Röntgen-Computertomographie", 16 March 2012 (2012-03-16), Universität Erlangen-Nürnberg, Erlangen,DE, XP055263288, * Section 2.3 * * page 69 * | 1-4 | |

| TECHNICAL FIELDS SEARCHED (IPC) |
|---|
| G01N G01T A61B |

The present search report has been drawn up for all claims

| Place of search | Date of completion of the search | Examiner |
|---|---|---|
| The Hague | 6 April 2016 | Steinmetz, Johannes |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 021 142 A1**

**REFERENCES CITED IN THE DESCRIPTION**

**Patent documents cited in the description**

- PL 213008

- PL 409092